# EUROPEAN PATENT APPLICATION

(11) **EP 1 985 700 A1**
(43) Date of publication of application: **29.10.2008**
(21) Application number: 07707937.4
(22) Date of filing: 02.02.2007
(51) Int. Cl.: C12N 15/00, C12N 1/21, C12N 9/04, C12N 15/09, C12P 7/02, C12P 41/00, C12R 1/01

(54) **NOVEL (S,S)-BUTANEDIOL DEHYDROGENASE, GENE FOR THE SAME, AND USE OF THE SAME**

(30) Priority: 14.02.2006 JP 2006037267
(71) Applicant: Kaneka Corporation, Kita-ku Osaka-shi, Osaka 530-8288 (JP)
(72) Inventor: MORIYAMA, Daisuke, Takasago-shi Hyogo 676-8688 (JP); TAOKA, Naoaki, Takasago-shi Hyogo 676-8688 (JP)
(74) Representative: Behnisch, Werner
(86) International application number: PCT/JP2007/051763
(87) International publication number: WO 2007/094178

(57) **Abstract**

The present invention has its object to provide a novel (S,S)-butanediol dehydrogenase. The present invention also has its object to provide a gene coding for the enzyme protein, a vector containing the gene, a transformant harboring the vector, and a method of producing an optically active alcohol using the transformant. The polypeptide according to one embodiment of the present invention has the physicochemical properties including actions such as: acting on (2S, 3S) -2, 3-butanediol to form (S) -acetoin in the presence of NAD⁺ as a coenzyme; and reducing 2,3-butanedione to form (2S, 3S) -2, 3-butanediol in the presence of NADH as a coenzyme.

SEQUENCE LISTING

<110> KANEKA CORPORATION

<120> NOVEL (S,S)-BUTANEDIOL DEHYDROGENASE, GENE FOR THE SAME, AND USE OF THE SAME

<130> B060005W001-

<140> PCT/JP2007/051763
<141> 2007-02-02

<150> JP2006-037267
<151> 2006-02-14

<160> 13

<170> PatentIn version 3.1

<210> 1
<211> 259
<212> PRT
<213> Ochrobactrum sp.

<400> 1

Met Ser Ala Asn Thr Lys Val Ala Leu Val Thr Gly Ala Ala Gln Gly
1               5                   10                  15

Ile Gly Arg Gly Ile Ala Leu Arg Leu Ala Lys Asp Gly Phe Asp Ile
            20                  25                  30

Ala Leu Val Asp Leu Lys Ala Asp Lys Leu Ala Ala Val Lys Lys Glu
        35                  40                  45

Val Glu Ala Leu Gly Arg Lys Ala Ser Thr Phe Ser Ala Asp Val Ser
    50                  55                  60

Lys Arg Asp Asp Val Tyr Ala Ala Ile Asp His Ala Glu Lys Glu Leu
65                  70                  75                  80

Gly Gly Phe Asp Val Ile Val Asn Asn Ala Gly Ile Ala Gln Val Gln
                85                  90                  95

Pro Ile Ala Asp Val Lys Pro Glu Glu Val Glu Arg Ile Phe Arg Ile
            100                 105                 110

Asn Val Asp Gly Val Leu Trp Gly Ile Gln Ala Ala Ala Ala Lys Phe
        115                 120                 125

Lys Ala Arg Lys Gln Lys Gly Lys Ile Ile Asn Ala Ser Ser Ile Ala
    130                 135                 140

Gly His Asp Gly Phe Ala Met Leu Gly Val Tyr Ser Ala Thr Lys Phe
145                 150                 155                 160

Ala Val Arg Ala Leu Thr Gln Ala Ala Ala Lys Glu Tyr Ala Ser Ala
                165                 170                 175

Gly Ile Thr Val Asn Ala Tyr Cys Pro Gly Ile Val Gly Thr Asp Met
            180                 185                 190

Trp Val Glu Ile Asp Glu Arg Phe Ser Glu Ile Thr Gly Ala Pro Lys
        195                 200                 205

Gly Glu Thr Tyr Lys Lys Tyr Val Asp Gly Ile Ala Leu Gly Arg Ala
    210                 215                 220

Gln Thr Pro Asp Asp Val Ala Ser Leu Val Ser Phe Leu Ala Ser Ala
225                 230                 235                 240

Asp Ser Asp Tyr Ile Thr Gly Gln Ala Ile Leu Thr Asp Gly Gly Ile
                245                 250                 255

Val Tyr Arg

<210> 2
<211> 780
<212> DNA
<213> Ochrobactrum sp.

<220>
<221> CDS
<222> (1)..(780)
<223>

<400> 2
atg tcg gct aac acc aag gtt gcg ctc gtt acc ggt gcg gca cag ggc 48
Met Ser Ala Asn Thr Lys Val Ala Leu Val Thr Gly Ala Ala Gln Gly
1               5                   10                  15

atc ggt cgg ggt att gcc ctt cgg ctg gcc aag gat ggt ttc gat atc 96
Ile Gly Arg Gly Ile Ala Leu Arg Leu Ala Lys Asp Gly Phe Asp Ile
            20                  25                  30

gct ctg gta gac ctc aag gcg gac aag ctc gcc gct gtc aaa aaa gag 144
Ala Leu Val Asp Leu Lys Ala Asp Lys Leu Ala Ala Val Lys Lys Glu
        35                  40                  45

gtc gaa gcc ttg ggc cgc aag gcg tcc aca ttc tca gcc gat gta agc 192
Val Glu Ala Leu Gly Arg Lys Ala Ser Thr Phe Ser Ala Asp Val Ser
    50                  55                  60

aag cgc gac gat gtt tac gcc gct atc gat cat gcg gaa aag gaa ctg 240
Lys Arg Asp Asp Val Tyr Ala Ala Ile Asp His Ala Glu Lys Glu Leu
65                  70                  75                  80

ggc ggc ttt gac gtg atc gtc aac aat gcc ggt atc gcg cag gtt cag 288
Gly Gly Phe Asp Val Ile Val Asn Asn Ala Gly Ile Ala Gln Val Gln
                85                  90                  95

ccg att gcg gat gtg aag ccg gaa gaa gtt gag cgt att ttc cgc atc 336
Pro Ile Ala Asp Val Lys Pro Glu Glu Val Glu Arg Ile Phe Arg Ile
            100                 105                 110

aat gtc gac ggt gtg ctc tgg ggt att cag gcg gct gcc gcc aag ttc 384
Asn Val Asp Gly Val Leu Trp Gly Ile Gln Ala Ala Ala Ala Lys Phe
        115                 120                 125

aag gcg cgc aag cag aag ggc aag atc atc aac gcc tcg tcc att gct 432
Lys Ala Arg Lys Gln Lys Gly Lys Ile Ile Asn Ala Ser Ser Ile Ala
    130                 135                 140

ggt cat gac ggc ttt gcc atg ctc ggc gtt tat tcc gcg aca aaa ttc 480
Gly His Asp Gly Phe Ala Met Leu Gly Val Tyr Ser Ala Thr Lys Phe
145                 150                 155                 160

gcc gtg cgt gcg ctg acg cag gct gcg gcc aag gaa tat gca agt gcg 528
Ala Val Arg Ala Leu Thr Gln Ala Ala Ala Lys Glu Tyr Ala Ser Ala
                165                 170                 175

ggc att acc gtg aac gct tat tgc ccc ggc atc gtc ggc acc gat atg 576
Gly Ile Thr Val Asn Ala Tyr Cys Pro Gly Ile Val Gly Thr Asp Met
            180                 185                 190

tgg gtc gaa atc gac gag cgg ttc tcc gag att acc ggc gcg ccg aag 624
Trp Val Glu Ile Asp Glu Arg Phe Ser Glu Ile Thr Gly Ala Pro Lys
        195                 200                 205

ggt gag acc tac aag aaa tat gtc gac ggc att gcg ctt ggt cgc gcc 672
Gly Glu Thr Tyr Lys Lys Tyr Val Asp Gly Ile Ala Leu Gly Arg Ala
    210                 215                 220

cag acg ccg gac gat gta gca tcc ctc gtt tcc ttc ctg gca agc gcg 720
Gln Thr Pro Asp Asp Val Ala Ser Leu Val Ser Phe Leu Ala Ser Ala
225                 230                 235                 240

gat tcc gat tac atc acg ggt cag gcc atc ctg acg gac ggc ggt atc 768
Asp Ser Asp Tyr Ile Thr Gly Gln Ala Ile Leu Thr Asp Gly Gly Ile
                245                 250                 255

gtt tac cgc tga                                                                780
Val Tyr Arg

<210>  3
<211>  20
<212>  DNA
<213>  Artificial

<220>
<223>  primer-1

<220>
<221>  misc_feature
<222>  (9)..(9)
<223>  n represents a, t, g or c.

<400>  3
aargayggnt tygayathgc                                                          20

<210>  4
<211>  20
<212>  DNA
<213>  Artificial

<220>

<223> primer-2

<220>

<221> misc_feature

<222> (6)..(6)

<223> n represents a, t, g or c.

<400> 4

gcytgnccyg tdatrtartc 20

<210> 5

<211> 628

<212> DNA

<213> Ochrobactrum sp.

<400> 5

tctggtagac ctcaaggcgg acaagctcgc cgctgtcaaa aaagaggtcg aagccttggg 60

ccgcaaggcg tccacattct cagccgatgt aagcaagcgc gacgatgttt acgccgctat 120

cgatcatgcg gaaaaggaac tgggcggctt tgacgtgatc gtcaacaatg ccggtatcgc 180

gcaggttcag ccgattgcgg atgtgaagcc ggaagaagtt gagcgtattt tccgcatcaa 240

tgtcgacggt gtgctctggg gtattcaggc ggctgccgcc aagttcaagg cgcgcaagca 300

gaagggcaag atcatcaacg cctcgtccat tgctggtcat gacggctttg ccatgctcgg 360

cgtttattcc gcgacaaaat tcgccgtgcg tgcgctgacg caggctgcgg ccaaggaata 420

tgcaagtgcg ggcattaccg tgaacgctta ttgccccggc atcgtcggca ccgatatgtg 480

ggtcgaaatc gacgagcggt tctccgagat taccggcgcg ccgaagggtg agacctacaa 540

gaaatatgtc gacggcattg cgcttggtcg cgcccagacg ccggacgatg tagcatccct 600

cgtttccttc ctggcaagcg cggattcc 628

<210> 6
<211> 24
<212> DNA
<213> Artificial

<220>
<223> primer-3

<400> 6
atcggctgag aatgtggacg cctt 24

<210> 7
<211> 24

<212> DNA
<213> Artificial

<220>
<223> primer-4

<400> 7
tatgtcgacg gcattgcgct tggt 24

<210> 8
<211> 35
<212> DNA
<213> Artificial

<220>
<223> primer-5

<400> 8
aggaaaggat gacatatgtc ggctaacacc aaggt 35

<210> 9
<211> 29
<212> DNA
<213> Artificial

<220>

<223>  primer-6

<400>  9
gcggaattct cagcggtaaa cgataccgc                                          29

<210>  10
<211>  43
<212>  DNA
<213>  Artificial

<220>
<223>  primer-7

<400>  10
gccgaattct aaggaggtta acaatgtcgg ctaacaccaa ggt                          43

<210>  11
<211>  29
<212>  DNA
<213>  Artificial

<220>
<223>  primer-8

<400>  11
gcgggatcct cagcggtaaa cgataccgc                                          29

<210> 12
<211> 43
<212> DNA
<213> Artificial

<220>
<223> primer-9

<400> 12
gccggatcct aaggaggtta acaatgtata aagatttaga agg 43

<210> 13
<211> 29
<212> DNA
<213> Artificial

<220>
<223> primer-10

<400> 13
gcgctgcagt tatccgcgtc ctgcttgga 29

## Description

### TECHNICAL FIELD

The present invention relates to a novel (S,S) -butanediol dehydrogenase. The invention also relates to a gene coding for the enzyme protein, a vector containing the gene, a transformant harboring the vector, and a method of producing an optically active alcohol using the transformant.

### BACKGROUND ART

(S,S)-Butanediol dehydrogenase is an enzyme playing an important role in biosynthesis and metabolism of 2, 3-butanediol or fermentative production of (2S, 3S) -2,3-butanediol starting from glucose. (2S,3S)-2,3-Butanediol, which is the product of this enzymatic reaction, is a compound useful as a raw material for the production of medicinal compounds and liquid crystals.

As (S, S) -butanediol dehydrogenase species which have been highly purified and characterized, there may be mentioned the following enzyme species.

- Zoogloea ramigera-derived (S,S)-butanediol dehydrogenase (Patent Document 1).
- Brevibacterium saccharolyticum C-1012-derived (S,S)-butanediol dehydrogenase (Non-Patent Document 1).
These two enzyme species both exert a selective oxidation activity only on (2S, 3S) -2,3-butanediol among the three isomers of 2,3-butanediol.
Patent Document 1: Japanese Kokai Publication 2004-357639 Non-Patent Document 1: Biosci. Biotechnol. Biochem., 65 (8), 1876-1878 (2001)

### SUMMARY OF THE INVENTION

It is an object of the present invention to provide a novel (S,S)-butanediol dehydrogenase species differing in substrate specificity from both the enzyme species disclosed in the documents cited above. Another object of the invention is to provide a gene coding for the enzyme protein, a vector containing the gene, a transformant harboring the vector, and a method of producing an optically active alcohol using the transformant.

[1] One characteristic of the present invention is a polypeptide having the physicochemical properties shown below under (1) to (2):
   (1) Actions:
      (a) Acting on (2S,3S)-2,3-butanediol to form (S)-acetoin in the presence of oxidized-form nicotinamide adenine dinucleotide (hereinafter abbreviated as NAD⁺) as a coenzyme;
      (b) Reducing 2,3-butanedione to form (2S,3S)-2,3-butanediol in the presence of reduced-form nicotinamide adenine dinucleotide (hereinafter abbreviated as NADH) as a coenzyme;
   (2) Substrate specificities:
      (a) Utilizing NAD⁺ as a coenzyme in oxidation reactions; also utilizing NADH as a coenzyme in reduction reactions;
      (b) Oxidizing not only the (S)-configuration hydroxyl group in (2S,3S)-2,3-butanediol among the three isomers of 2,3-butanediol but also the (S)-configuration hydroxyl group in meso-2,3-butanediol.
[2] One characteristic of the present invention is the polypeptide according to [1], further having the physicochemical property shown below under (3):
   (3) Substrate specificities: Selectively oxidizing the (R)-configuration hydroxyl group in racemic 3-chloro-1,2-propanediol and allowing (S)-3-chloro-1,2-propanediol to continue to exist.
[3] One characteristic of the present invention is the polypeptide according to [1] or [2], further having the physicochemical properties shown below under (4) to (6): (4) Molecular weight: about 30,000 as determined by sodium dodecyl sulfate-polyacrylamide gel electrophoresis;
   (5) Optimum pH for oxidation: from 10.0 to 12.0;
   (6) Optimum pH for reduction: from 5.0 to 5.5.
[4] One characteristic of the present invention is the polypeptide according to any of [1] to [3], which is produced by a microorganism belonging to the genus Ochrobactrum.
[5] One characteristic of the present invention is the polypeptide according to [4], wherein the microorganism belonging to the genus Ochrobactrum is an Ochrobactrum sp.
[6] One characteristic of the present invention is a polypeptide defined below under (a), (b) or (c):
   (a) a polypeptide comprising the amino acid sequence shown in the sequence listing under SEQ ID NO:1;
   (b) a polypeptide comprising an amino acid sequence derived from the amino acid sequence shown in the sequence listing under SEQ ID NO:1 by substitution, insertion, deletion and/or addition of one or a plurality of amino acids, the polypeptide having an enzymatic activity of selectively oxidizing the (R)-configuration hydroxyl group of racemic 3-chloro-1,2-propanediol and allowing (S)-3-chloro-1,2-propanediol to continue to exist;
   (c) a polypeptide comprising an amino acid sequence having at least 80% sequence homology to the amino acid sequence shown in the sequence listing under SEQ ID NO:1, the polypeptide having an enzymatic activity of selectively oxidizing the (R)-configuration hydroxyl group in racemic 3-chloro-1,2-propanediol and allowing (S)-3-chloro-1,2-propanediol to continue to exist.
[7] One characteristic of the present invention is a DNA coding for the polypeptide according to any of [1] to [6].
[8] One characteristic of the present invention is a DNA defined below under (a), (b) or (c):
   (a) a DNA comprising the base sequence shown in the sequence listing under SEQ ID NO:2;
   (b) a DNA capable of hybridizing with a DNA comprising a base sequence complementary to the base sequence shown in the sequence listing under SEQ ID NO:2 under stringent conditions, the DNA coding for a polypeptide having an enzymatic activity of selectively oxidizing the (R)-configuration hydroxyl group in racemic 3-chloro-1,2-propanediol and allowing (S)-3-chloro-1,2-propanediol to continue to exist;
   (c) a DNA comprising a base sequence having at least 80% sequence homology to the base sequence shown in the sequence listing under SEQ ID NO:2, the DNA coding for a polypeptide having an enzymatic activity of selectively oxidizing the (R)-configuration hydroxyl group in racemic 3-chloro-1,2-propanediol and allowing (S)-3-chloro-1,2-propanediol to continue to exist.
[9] One characteristic of the present invention is an expression vector comprising the DNA according to [7] or [8].
[10] One characteristic of the present invention is the expression vector according to [9], which is the plasmid pTSOB capable of being isolated from Escherichia coli HB101 (pTSOB) (FERM BP-10461).
[11] One characteristic of the present invention is a transformant obtained by transforming host cells with the expression vector according to [9] or [10].
[12] One characteristic of the present invention is the transformant according to [11], wherein the host cell is Escherichia coli.
[13] One characteristic of the present invention is the transformant according to [11], which is Escherichia coli HB101 (pTSOB) (FERM BP-10461).
[14] One characteristic of the present invention is a method of producing an optically active alcohol, comprising the steps of:
   allowing the polypeptide or the culture product of the transformant to act on a racemic alcohol to thereby oxidize an alcohol having one configuration and allow an alcohol having the other configuration to continue to exist; and
   collecting the optically active alcohol which is allowed to remain.
[15] One characteristic of the present invention is a method of producing an optically active alcohol, comprising the steps of:
   allowing the polypeptide or the culture product of the transformant to react with a compound having a carbonyl group; and
   collecting the optically active alcohol formed.

The present invention provides a novel (S,S)-butanediol dehydrogenase species, a DNA coding for the enzyme, and a transformant harboring the DNA. The invention also provides a practical method of producing optically active useful alcohols, such as (S)-3-chloro-1,2-propanediol, using the enzyme and the transformant.

### BRIEF DESCRIPTION OF THE DRAWING

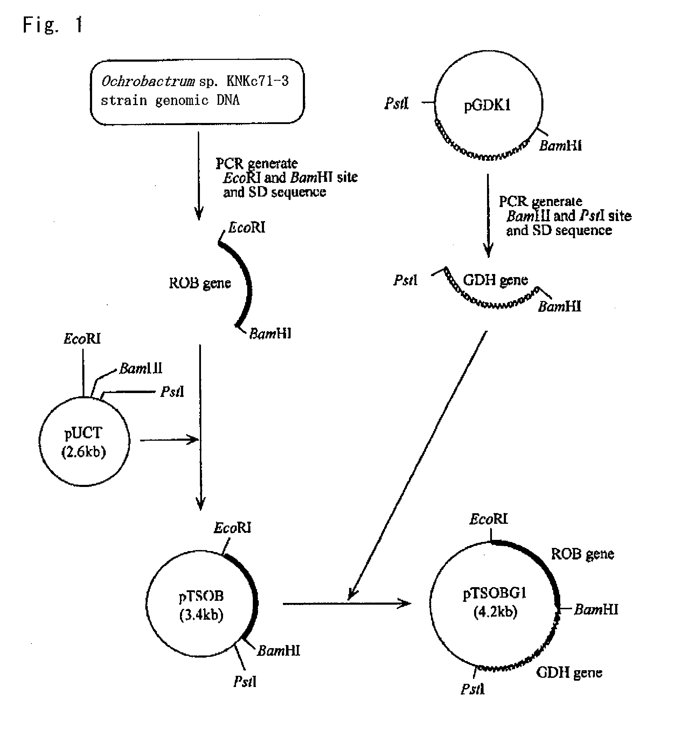

Fig. 1 is a schematic representation of processes for constructing the recombinant vectors pTSOB and pTSOBG1.

### DETAILED DESCRIPTION OF THE INVENTION

In the following, the present invention is described in detail referring to typical modes of embodiment thereof. It is to be noted that these modes of embodiment are by no means limitative of the scope of the invention.

The gene manipulation procedures described herein, for example DNA isolation, vector preparation and transformation, can be carried out as described in such monographs as Molecular Cloning 2nd Edition (Cold Spring Harbor Laboratory Press, 1989) and Current Protocols in Molecular Biology (Greene Publishing Associates and Wiley-Interscience), unless otherwise specifically mentioned. The symbol "%" as used herein means "% (w/v)", unless otherwise specified.

### 1. Polypeptide

In the practice of the invention, the "polypeptide" can be obtained from a microorganism capable of oxidizing the hydroxyl group in (R) configuration of racemic 3-chloro-1,2-propanediol while allowing (S)-3-chloro-1,2-propanediol to remain. The microorganism to be used as the source of the polypeptide is not particularly restricted but mention may be made of bacteria which belong to the genus Ochrobactrum; the Ochrobactrum sp. KNKc71-3 strain can be mentioned as a particularly preferred one. The KNKc71-3 strain is a microorganism isolated from soil by the present inventors.

Typical bacteriological properties of the Ochrobactrum sp. KNKc71-3 strain are shown below.
A. Morphology
   (1) Rods with a cell size of 0.7 to 0.8 x 1.5 to 2.0 µm.
   (2) Flagellar motility.
   (3) No spore.
   (4) Gram stain: negative.
   (5) Colony morphology: circular, convex, entire edges smooth, cream-colored.
B. Physiological characteristics
   (1) Reduction of nitrate: negative.
   (2) Indole production: negative.
   (3) Glucose acidification: negative.
   (4) Arginine dihydrolase activity: negative.
   (5) Urease activity: negative.
   (6) Esculin hydrolysis: negative.
   (7) Gelatin hydrolysis: negative.
   (8) β-Galactosidase activity: negative.
   (9) Oxidase activity: positive.
   (10) Catalase activity: positive.
   (11) Assimilation test: assimilable are glucose, L-arabinose, D-mannose, N-acetyl-D-glucosamine, maltose, dl-malic acid and sodium citrate.
   (12) Growth on MacConkey agar: growth occurs.
   (13) Tween 80 hydrolysis: negative.
   (14) Growth under anaerobic conditions: no growth.
The microorganism producing the polypeptide (enzyme) according to the invention may be a wild type one or a mutant type one. A microorganism derived by such a genetic technique as cell fusion or gene manipulation, which is well known in the art, may also be used. Such a genetically engineered microorganism can be obtained, for example, by the process well known in the art, including the steps of: isolating and/or purifying such enzyme and determining a part or the whole of the amino acid sequence of the enzyme; determining the base sequence of a DNA coding for the polypeptide based on that amino acid sequence; obtaining a DNA coding for the polypeptide based on that base sequence; obtaining a recombinant microorganism by introducing that DNA into an appropriate host microorganism; and cultivating the recombinant microorganism to obtain the enzyme according to the invention.

As the polypeptide according to the invention, there may be mentioned, for example, polypeptides having the following physicochemical properties (1) and (2):
(1) Actions:
   (a) Acting on (2S,3S)-2,3-butanediol, with oxidized-form nicotinamide adenine dinucleotide (hereinafter abbreviated as NAD⁺) as a coenzyme, to form (S)-acetoin.
   (b) Reducing 2,3-butanedione, with reduced-form nicotinamide adenine dinucleotide (hereinafter abbreviated as NADH) as a coenzyme, to form (2S,3S)-2,3-butanediol.
(2) Substrate specificities:
   (a) Utilizing NAD⁺ as a coenzyme in oxidation reactions. Utilizing NADH as a coenzyme in reduction reactions.
   (b) Oxidizing not only the (S)-configuration hydroxyl group in (2S,3S)-2,3-butanediol but also the (S)-configuration hydroxyl group in meso-2,3-butanediol among the three 2,3-butanediol isomers.

There may preferably be mentioned polypeptides having the following physicochemical property (3) in addition to the above physicochemical properties (1) and (2):
(3) Substrate specificity: Selectively oxidizing the (R)-configuration hydroxyl group in racemic 3-chloro-1,2-propanediol while allowing (S)-3-chloro-1,2-propanediol to continue to exist.

More preferably, there may be mentioned polypeptides having the physicochemical properties given below under (4) to (6) in addition to the above physicochemical properties (1) to (3):
(4) Molecular weight: about 30,000 as determined by sodium dodecyl sulfate-polyacrylamide gel electrophoresis.
(5) Optimum pH for oxidation: 10.0 to 12.0.
(6) Optimum pH for reduction: 5.0 to 5.5.

Further, the polypeptide may be a polypeptide comprising the amino acid sequence shown in the sequence listing under SEQ ID NO:1, or a polypeptide comprising the amino acid sequence derived from the amino acid sequence shown in the sequence listing under SEQ ID NO:1 by substitution, insertion, deletion and/or addition of one or a plurality of (e.g. 20, preferably 15, more preferably 10, still more preferably 5, 4, 3, 2, or less) amino acids and enzymatically capable of selectively oxidizing the (R)-configuration hydroxyl group in racemic 3-chloro-1,2-propanediol while allowing (S)-3-chloro-1,2-propanediol to continue to exist.

Those polypeptides comprising an amino acid sequence derived from the amino acid sequence shown in the sequence listing under SEQ ID NO:1 by substitution, insertion, deletion and/or addition of one or a plurality of amino acids can be prepared such methods known in the art as those described in Current Protocols in Molecular Biology (John Wiley and Sons, Inc., 1989) and, so long as they are enzymatically active in selectively oxidizing the (R)-configuration hydroxyl group in racemic 3-chloro-1,2-propanediol while allowing (S)-3-chloro-1,2-propanediol to continue to exist, they are included under the polypeptide category mentioned above.

In the amino acid sequence shown in the sequence listing under SEQ ID NO:1, the site or sites at which amino acid substitution, insertion, deletion and/or addition is to be carried out are not particularly restricted but highly conserved regions are preferably avoided. The highly conserved regions, so referred to herein, are domains identical in amino acid sequence among a plurality of sequences when the amino acid sequences of a plurality of enzymes differing in origin are optimally arranged in parallel for comparison. Such highly conserved regions can be identified by comparing the amino acid sequence shown under SEQ ID NO:1 with the amino acid sequences of such other microorganism-derived 2,3-butanediol dehydrogenases as mentioned above using GENETYX or a like tool.

The amino acid sequence modified by substitution, insertion, deletion and/or addition may comprise only one type of modification (for example, substitution) or two or more types of modification (for example, substitution and insertion).

In the case of substitution, the amino acid or acids after substitution are preferably homologous with the original amino acids. Those amino acids which belong to the same group out of the four groups given below are regarded as homologous with one another or each other.
(First group: neutral nonpolar amino acids) Gly, Ala, Val, Leu, Ile, Met, Cys, Pro, Phe
(Second group: neutral polar amino acids) Ser, Thr, Gln, Asn, Trp, Tyr
(Third group: acidic amino acids) Glu, Asp (Fourth group: basic amino acids) His, Lys, Arg
The number of amino acids to be substituted, inserted, deleted and/or added is not particularly restricted provided that the polypeptide after modification has an enzymatic activity such that it selectively oxidizes the (R)-configuration hydroxyl group in racemic 3-chloro-1,2-propanediol while allowing (S)-3-chloro-1,2-propanediol to continue to exist. Preferably the polypeptide after modification has at least 80% sequence homology, more preferably at least 90% sequence homology, still more preferably at least 95% sequence homology, most preferably at least 99% sequence homology, with the amino acid sequence shown in the sequence listing under SEQ ID NO:1. The sequence homology is given as the value obtained by dividing the number of identical sites found upon comparison, in the same manner as in the above-mentioned highly conserved region identification, between the amino acid sequence shown in the sequence listing under SEQ ID NO:1 and the modified amino acid sequence by the total number of amino acids compared and multiplying the quotient by 100.

### 2. Enzyme purification

In the following, an exemplary method of obtaining the polypeptide according to the invention from the Ochrobactrum sp. KNKc71-3 strain is described. This example is, however, by no means limitative of the scope of the invention.

First, a microorganism having an enzyme activity such that it selectively oxidizes the (R)-configuration hydroxyl group in racemic 3-chloro-1,2-propanediol while allowing (S)-3-chloro-1,2-propanediol to continue to exist is cultivated on an appropriate medium. As for the method of cultivation, a liquid nutrient medium containing ordinary carbon and nitrogen sources, inorganic salts and organic nutrients, among others, can be used provided that the microorganism can grow thereon. The cultivation can be carried out, for example, at a temperature of 25°C to 37°C and at a pH of 4 to 8, with shaking or aeration.

After separation of cells from the culture fluid by centrifugation, the cells are suspended in an appropriate buffer and disrupted or lysed using such a physical means as glass beads or a biochemical means as an enzyme, for instance. Further, the solid matter in the resulting solution is removed by centrifugation, whereby a crude enzyme solution can be obtained. The crude enzyme solution can be further purified by using one or a combination of those techniques which those skilled in the art generally use, for example precipitation with ammonium sulfate, dialysis and chromatography. As for the chromatography, hydrophobic chromatography, ion exchange chromatography and gel filtration chromatography may be used singly or in combination.

In the practice of the invention, the oxidation activity of the enzyme can be determined by adding a substrate (0.1 M), NAD⁺ (2 mM) and an enzyme solution to 100 mM phosphate buffer (pH 8.0) and measuring the increase in absorbance at the wavelength 340 nm at 30°C. The reduction activity of the enzyme can be determined by adding a substrate (20 mM), NADH (0.167 mM) and an enzyme solution to 100 mM phosphate buffer (pH 5.5) and measuring the decrease in absorbance at the wavelength 340 nm at 30°C.

The optimum pH and optimum temperature for the enzyme can be determined, for example, by varying the reaction pH and reaction temperature in the above activity measurement systems and measuring the activities. The molecular weight of the enzyme can be determined, for example, by gel filtration analysis using a Superdex 200HR10/30 (product of Pharmacia Biotech) column, followed by calculation based on the relative elution times of standard proteins. The subunit molecular weights can be determined by 20% SDS-polyacrylamide gel electrophoresis, followed by calculation based on the relative mobilities of standard proteins.

### 3. DNA

The "DNA" according to the invention is a DNA coding for the above-mentioned polypeptide and may be any one capable of expressing the polypeptide in host cells harboring the DNA introduced therein by such a method as described later herein. It may contain one or more untranslated regions. The DNA according to the invention can be obtained from a microorganism serving as a source of the polypeptide by the methods known to those skilled in the art, so long as the polypeptide can be obtained from the microorganism.

An exemplary method of obtaining a DNA coding for the enzyme according to the invention is described below. This example is, however, by no means limitative of the scope of the invention.

First, the purified enzyme is digested with an appropriate endopeptidase, a cleaved fragment is purified by reversed phase HPLC using a YMC-Pack SIL-06 (product of YMC) column, and the partial amino acid sequence is partially determined using a model AB 1492 protein sequencer (product of Applied Biosystems) . Base on the partial amino acid sequence, PCR primers are synthesized. Then, the chromosomal DNA is prepared from the microorganism serving as the source of the DNA by an ordinary method of DNA isolation, for example the Hereford method (Cell, 18, 1261 (1979)). PCR is carried out using this chromosomal DNA as the template, together with the above-mentioned PCR primers for amplification of a part (core sequence) of the DNA coding for the polypeptide, and the base sequence of that part is determined. The base sequence can be determined, for example, by the dideoxy chain termination method, which can be carried out by using a model ABI 373A DNA sequencer (product of Applied Biosystems), for example.

For revealing the base sequence of the regions neighboring the core sequence, the chromosomal DNA of the microorganism is digested with a restriction enzyme the recognition sequence of which is absent in the core sequence and the DNA fragment formed is treated with T4 ligase for self-circularization to thereby prepare a template DNA for inverse PCR (Nucleic Acids Res., 16, 8186 (1988)). Then, based on the core sequence, primers to serve as starting points for DNA synthesis in the outward directions from the core sequence are synthesized, and the regions neighboring the core sequence are amplified by inverse PCR. By clarifying the base sequence of the thus-obtained DNA, it becomes possible to reveal the DNA sequence of the whole coding region of the target enzyme.
As the DNA according to the invention, there may be mentioned, for example, a DNA comprising the base sequence shown in the sequence listing under SEQ ID NO:2 or a DNA capable of hybridizing with a DNA comprising a base sequence complementary to the base sequence shown in the sequence listing under SEQ ID NO:2 under stringent conditions.

The "DNA capable of hybridizing with a DNA comprising a base sequence complementary to the base sequence shown in the sequence listing under SEQ ID NO:2 under stringent conditions", so referred to herein, means a DNA obtained by using a DNA comprising a base sequence complementary to the base sequence shown in the sequence listing under SEQ ID NO:2 as a probe and carrying out the colony hybridization, plaque hybridization or Southern hybridization method, for instance, under stringent conditions.

The hybridization can be carried out by the methods described in Molecular Cloning, A Laboratory Manual, second edition (Cold Spring Harbor Laboratory Press, 1989), for instance. As the "DNA capable of hybridizing under stringent conditions", there may be mentioned, for example, a DNA obtained by carrying out the hybridization at 65°C in the presence of 0.7 to 1.0 M NaCl using a filter with a colony- or plaque-derived DNA immobilized thereon and then washing the filter with a twice concentrated SSC solution (unit concentration SSC solution having a composition of 150 mM sodium chloride and 15 mM sodium citrate) at 65°C, preferably with a 1/2 concentrated SSC solution at 65°C, more preferably with a 1/5 concentrated SSC solution at 65°C, still more preferably with a 1/10 concentrated SSC solution at 65°C.

As the DNA capable of hybridizing under the conditions mentioned above, there may be mentioned a DNA having at least 60%, preferably at least 80%, more preferably at least 90%, still more preferably at least 95%, most preferably at least 99%, sequence homology to the DNA shown under SEQ ID NO:2 and, so long as the polypeptide encoded by a DNA has an activity such that it selectively oxidizes the (R)-configuration hydroxyl group in racemic 3-chloro-1,2-propanediol while allowing (S)-3-chloro-1,2-propanediol to continue to exist, the DNA falls within the DNA category defined above.

The "sequence homology (%)", so referred to herein, is given as the numerical value obtained by arranging two DNAs to be compared with each other in parallel in an optimal manner, dividing the thus-found number of sites coincident in nucleic acid base (e.g. A, T, C, G, U or I) between both sequences by the total number of bases compared and multiplying the quotient by 100.

The sequence homology can be calculated using, for example, the following sequence analyzing tools: GCG Wisconsin Package (Program Manual for The Wisconsin Package, Version 8, September 1994, Genetics Computer Group, 575 Science Drive Madison, Wisconsin, USA 53711; Rice, P. (1996) Program Manual for EGCG Package, Peter Rice, The Sanger Centre, Hinxton Hall, Cambridge, CB10 1RQ, England); and the ExPASy World Wide Web server for molecular biology (Geneva University Hospital and University of Geneva, Geneva, Switzerland).

### 4. Vector

Usable as the vector DNA to be used in introducing the enzyme DNA according to the invention into a host microorganism and causing the expression thereof in the host microorganism are any of the vector DNAs capable of allowing the enzyme gene to be expressed in an appropriate host microorganism. As such vector DNAs, there may be mentioned, for example, plasmid vectors, phage vectors and cosmid vectors. Shuttle vectors capable of gene exchange with another host strain can also be used.

Such a vector comprises such a regulatory factor of an operably connected promoter (lacUV5 promoter, trp promoter, trc promoter, tac promoter, lpp promoter, tufB promoter, recA promoter, pL promoter, etc.) and can be suitably used as an expression vector comprising an expression unit operably connected with the DNA according to the invention. For example, pUCNT (WO 94/03613) can be suitably used.

The term "regulatory factor" as used herein means a base sequence containing a functional promoter and any related transcription elements (e.g. enhancer, CCAAT box, TATA box, SPI site).

The phrase "operably connected" as used herein means that the DNA is connected with various regulatory elements, such as a promoter or enhancer, which controls the expression of that DNA, so that the regulatory elements may operate in host cells. As is well known to those skilled in the art, the type and species of the regulatory factor may vary according to the host.

### 5. Host cells

As the "host cells" into which a vector containing the DNA according to the invention is to be introduced, there may be mentioned bacterial cells, yeast cells, filamentous fungal cells, plant cells and animal cells, among others. From the introduction and expression efficiency points of view, however, bacterial cells are preferred and Escherichia coli cells are particularly preferred. The DNA according to the invention can be introduced into host cells in the conventional manner. In cases where Escherichia coli cells are used as host cells, a vector containing the DNA according to the invention can be introduced into the host cells by the calcium chloride method, for instance.

### 6. Transformant

The "transformant" according to the invention can be obtained by inserting the above-mentioned polypeptide-encoding DNA into the above-mentioned vector and introducing the resulting expression vector into host cells. The transformant can be cultivated using a liquid nutrient medium containing ordinary carbon source(s), nitrogen source(s), inorganic salts and organic nutrients, among others, provided that the transformant can grow therein.

As an example of the transformant mentioned above, there may be mentioned E. coli HB101 (pTSOB), which is to be described later herein. This transformant has been deposited as of November 30, 2005 with the National Institute of Advanced Industrial Science and Technology International Patent Organism Depositary (zip code 305-8566, Chuo No. 6 Higashi 1-1-1 Tsukuba City, Ibaraki Prefecture) under the accession number FERM BP-10461.

### 7. Method of producing optically active alcohols

There is now described a method of producing optically active alcohols by allowing the enzyme according to the invention or a transformant capable of producing the enzyme to act on a racemic alcohol to thereby oxidize the alcohol having either one of the configurations preferentially while allowing the alcohol having the other configuration to continue to exist.

In allowing the above-mentioned enzyme or transformant to act on a racemic alcohol and preferentially oxidizing the alcohol having one of the configurations, NAD⁺ is required as a coenzyme. The reaction can be carried out by adding a necessary amount of NAD⁺ to the reaction system. However, by carrying out the reaction using an enzyme having an ability to convert the reduced coenzyme (NADH) to the oxidized form (NAD⁺) (hereinafter referred to as oxidized-form coenzyme regenerating ability), together with a substrate therefor, namely by carrying out the reaction using the enzyme according to the invention in combination with an oxidized-form coenzyme regenerating system, it becomes possible to markedly reduce the amount of the coenzyme to be used.

Usable as the enzyme having oxidized-form coenzyme regenerating ability are, for example, NADH oxidase and NADH dehydrogenase. While such a reaction may also be carried out by adding an oxidized-form coenzyme regenerating system to the asymmetric oxidation reaction system, it becomes possible to carry out the reaction efficiently, without separately preparing an enzyme having oxidized-form coenzyme regenerating ability and adding the same to the reaction system, when a transformant as transformed with both a DNA coding for the above-mentioned enzyme and a DNA coding for NADH oxidase is used as a catalyst. Such a transformant can be obtained by inserting a DNA coding for the enzyme and a DNA coding for NADH oxidase into one and the same vector and introducing the resulting expression vector into host cells or by inserting these two DNAs respectively into two vector species differing in incompatibility group and introducing the resulting expression vectors into the same host cells.

In cases where the transformant according to the invention is used, the reaction proceeds in the presence of NAD⁺ occurring in bacterial cells and the NADH formed upon reduction of NAD⁺ is again oxidized in the cells; therefore, the reaction can be carried out without separately adding the coenzyme and oxidized-form coenzyme regenerating system.

The production of optically active alcohols from racemic alcohols using the culture product of the enzyme or transformant according to the invention can be carried out in the following manner. The following method has no limitative meaning, however.

First, a racemic alcohol serving as a substrate, a coenzyme such as NAD⁺ and the transformant culture product are added to an appropriate solvent and the reaction is allowed to proceed with pH adjustment and stirring. The solvent to be used in carrying out the reaction may be an aqueous solvent or a mixture of an aqueous system and an organic solvent. As the organic solvent, there may be mentioned, for example, toluene, ethyl acetate, n-butyl acetate, hexane, isopropanol, diisopropyl ether, methanol, acetone, and dimethyl sulfoxide. This reaction is carried out at a temperature of 10 to 70°C, and the pH of the reaction mixture is kept at 6 to 12 during the reaction. The reaction may be carried out batchwise or continuously. In the case of batchwise operation, the reaction substrate may be added at a charge concentration of 0.1% to 70% (w/v).

The term "culture product" as used herein indicates microbial cells, a culture fluid containing cells or a product derived from cells by some treatment. As the cell treatment product, there may be mentioned, for example, dried cells obtained by dehydrating treatment using acetone or diphosphorus pentaoxide or drying utilizing a desiccator or fan, surfactant treatment products, lytic enzyme treatment products, immobilized cells, and cell-free extract preparations resulting from disruption of cells. Further, it is also possible to purify the enzyme according to the invention from the culture and use the same.

When, in carrying out the reaction, a transformant capable of producing both the enzyme according to the invention and NADH oxidase is used, it is possible to markedly reduce the coenzyme amount by feeding oxygen to the reaction system.
The method of collecting the optically active alcohol obtained by any of the methods described above is not particularly restricted. For example, the optically active alcohol can be obtained in a highly pure form with ease by extracting the same directly from the reaction mixture or from the reaction mixture after separation of cells and so forth with such a solvent as ethyl acetate, toluene, t-butyl methyl ether or hexane and, after dehydration, purifying the same by distillation, crystallization or silica gel column chromatography, for instance.
Now, the method of producing optically active alcohols by allowing the enzyme according to the invention or a transformant capable of producing that enzyme to act on a carbonyl compound is described in detail.

In cases where an optically active alcohol is obtained by stereoselectively reducing a carbonyl group-containing compound using the above-mentioned enzyme or transformant, NADH is required as a coenzyme. A necessary amount of NADH may be added to the reaction system for carrying out the reaction. However, by using an enzyme having an ability to convert the oxidized coenzyme (NAD⁺) to the reduced form (NADH) (hereinafter referred to as reduced-form coenzyme regenerating ability), together with a substrate therefor, namely by carrying out the reaction using a reduced-form coenzyme regenerating system in combination with the enzyme according to the invention, it becomes possible to markedly reduce the amount of the coenzyme to be used.

Usable as the enzyme having reduced-form coenzyme regenerating ability are hydrogenase, formate dehydrogenase, alcohol dehydrogenase, glucose-6-phosphate dehydrogenase and glucose dehydrogenase, among others. Preferably used are glucose dehydrogenase and formate dehydrogenase. While such a reaction may also be carried out by adding a reduced-form coenzyme regenerating system to the asymmetric reduction reaction system, it becomes possible to carry out the reaction efficiently, without separately preparing an enzyme having reduced-form enzyme regenerating ability and adding the same to the reaction system, when a transformant as transformed with a DNA coding for the enzyme mentioned above and a DNA coding for glucose dehydrogenase is used as a catalyst. Such a transformant can be obtained by inserting a DNA coding for the above-mentioned enzyme and a DNA coding for glucose dehydrogenase into one and the same vector and introducing the resulting expression vector into host cells, or by inserting these two DNAs respectively into two vector species differing in incompatibility group and introducing the resulting vectors into the same host cells.

The production of optically active alcohols from carbonyl group-containing compounds using the culture product of the enzyme or transformant according to the invention can be carried out in the following manner. The following method has no limitative meaning, however.

First, a carbonyl group-containing compound serving as a substrate, a coenzyme such as NADH and the transformant culture product are added to an appropriate solvent and the reaction is allowed to proceed with pH adjustment and stirring. The solvent to be used in carrying out the reaction may be an aqueous solvent or a mixture of an aqueous system and an organic solvent. As the organic solvent, there may be mentioned, for example, toluene, ethyl acetate, n-butyl acetate, hexane, isopropanol, diisopropyl ether, methanol, acetone and dimethyl sulfoxide. This reaction is carried out at a temperature of 10 to 70°C, and the pH of the reaction mixture is kept at 4 to 10 during the reaction. The reaction may be carried out batchwise or continuously. In the case of batchwise operation, the reaction substrate may be added at a charge concentration of 0.1% to 70% (w/v).

When, in carrying out the reaction, a transformant capable of producing both the enzyme according to the invention and glucose dehydrogenase is used, it is possible to markedly reduce the coenzyme amount by further adding glucose to the reaction system.
The method of collecting the optically active alcohol obtained by any of the methods described above is not particularly restricted. For example, the optically active alcohol can be obtained in a highly pure form with ease by extracting the same directly from the reaction mixture or from the reaction mixture after separation of cells and so forth with such a solvent as ethyl acetate, toluene, t-butyl methyl ether or hexane and, after dehydration, purifying the same by distillation, crystallization or silica gel column chromatography, for instance.

### BEST MODE FOR CARRYING OUT THE INVENTION

The following examples illustrate the present invention in detail. They are, however, by no means limitative of the scope of the present invention.

### (Example 1) Purification of (S,S)-butanediol dehydrogenase

In the examples described below, the oxidation activity of (S,S)-butanediol dehydrogenase was determined by adding (2S,3S)-2,3-butanediol (100 mM), NAD⁺ (2 mM) and an enzyme solution to 100 mM phosphate buffer (pH 8.0) and measuring the increase in absorbance at the wavelength 340 nm at 30°C. The enzyme activity reducing 1 µmol of NAD⁺ to NADH per minute was defined as 1 unit.

A liquid medium (pH 6.0) containing 1% of meat extract, 1.5% of polypeptone, 0.5% of Bacto yeast extract and 0.3% of NaCl was distributed, in 100 ml portions, into 500 ml Sakaguchi flasks, and steam-sterilized at 120°C for 20 minutes. This liquid medium was aseptically inoculated with the Ochrobactrum sp. KNKc71-3 strain and shake culture was carried out at 30°C for 40 hours. Cells were collected from the thus-obtained culture fluid (3 L) by centrifugation, washed with 500 ml of 10 mM phosphate buffer (pH 7.0) and suspended in 200 ml of 10 mM phosphate buffer (pH 7.0). The cells thus suspended were ultrasonically disrupted using SONFIER 250 (product of Branson), cell debris were removed by centrifugation, and a cell-free extract was obtained.

Ammonium sulfate was added to this cell-free extract to 30% saturation, the mixture was stirred at 4°C for 30 minutes and, then, the precipitate formed was removed by centrifugation. Further, ammonium sulfate was added to the supernatant to 75% saturation and, after 30 minutes of stirring at 4°C, the precipitate formed was recovered by centrifugation, suspended in 10 mM phosphate buffer (pH 7.0) and dialyzed against 10 mM phosphate buffer (pH 7.0).

The enzyme solution after dialysis was submitted to a DEAE-TOYOPEARL 650M (product of Tosoh) column (400 ml) equilibrated in advance with 10 mM phosphate buffer (pH 7.0) and a (S, S) -butanediol dehydrogenase-active fraction which had not been adsorbed on the column (flow-through fraction) was recovered.

Ammonium sulfate was added to this active fraction to a concentration of 1 M, and the mixture was submitted to a Phenyl-TOYOPEARL 650M (product of Tosoh) column (50 ml) equilibrated in advance with 10 mM phosphate buffer (pH 7.0) containing 1 M ammonium sulfate for adsorption of the enzyme. After washing the column with the same buffer, the active fraction was eluted on a linear gradient from 1 M to 0 M ammonium sulfate. Ammonium sulfate was added to this active fraction to a concentration of 1.2 M, and the mixture was submitted to a Butyl-TOYOPEARL 650S (product of Tosoh) column (25 ml) equilibrated in advance with 10 mM phosphate buffer (pH 7.0) containing 1.2 M ammonium sulfate for adsorption of the enzyme. After washing the column with the same buffer, the active fraction was eluted on a linear gradient from 1.2 M to 0 M ammonium sulfate. This active fraction was analyzed by sodium dodecyl sulfate-polyacrylamide electrophoresis and found to give a single band.

The purified enzyme has a specific activity of about 119 U/mg-protein. Hereinafter, this enzyme is referred to as ROB. The history of purification was as shown in Table 1.

**[Table 1]**

| | Protein amount | Total activity | Specific activity |
|---|---|---|---|
| | (mg) | (U) | (U/mg) |
| Cell-free extract | 4931 | 5585 | 1.12 |
| Ammonium sulfate fractionation | 3752 | 4894 | 1.30 |
| DEAE TOYOPEARL 650M | 176 | 1915 | 10.9 |
| Phenyl TOYOPEARL 650M | 6.8 | 688 | 101 |
| Butyl TOYOPEARL 650S | 5.1 | 610 | 119 |

### (Example 2) Measurement of molecular weight of ROB

The subunit molecular weight of the purified ROB obtained in Example 1 was determined by sodium dodecyl sulfate-polyacrylamide electrophoresis and was found to be about 30,000. As a result of analysis of ROB by gel filtration chromatography on Superdex 200HR10/30 (product of Pharmacia Biotech), the molecular weight of this enzyme as calculated based on the retention time relative to those of standard proteins was found to be about 101,000.

### (Example 3) Optimum temperature for action of ROB

Using the purified ROB obtained in Example 1, (2S,3S)-2,3-butanediol oxidation activity measurements were carried out at 10 to 70°C. In the activity measurements, (2S,3S)-2,3-butanediol (0.1 M), NAD⁺ 2 mM and the enzyme solution were added to 100 mM phosphate buffer (pH 8.0), the reaction was allowed to proceed at 10 to 70°C for 1 minute, and the increase in absorbance at the wavelength 340 nm were measured. As a result, the optimum temperature for the activity was found to be 70°C.

### (Example 4) Optimum pH in reduction reaction using ROB

Using the purified ROB obtained in Example 1, 2,3-butanedione reduction activity measurements were carried out at pH 4 to 9. In the activity measurements, 2,3-butanedione (10 mM), NADH (0.167 mM) and the enzyme solution were added to each buffer, the reaction was allowed to proceed at 30°C for 3 minutes, and the decrease in absorbance at the wavelength 340 nm was measured. The reduction activity measurements were performed within the pH range of 4 to 9 using, as the buffers, 100 mM citrate buffers (pH 4 to 6), 100 mM phosphate buffers (pH 5 to 8) and 100 mM Tris-HCl buffers (pH 8 to 9). As a result, the optimum pH for the reduction was found to be 5.0 to 5.5.

### (Example 5) Optimum pH in oxidation reaction using ROB

Using the purified ROB obtained in Example 1, (2S,3S)-2,3-butanediol oxidation activity measurements were carried out at pH 6 to 12. In the activity measurements, (2S,3S)-2,3-butanediol (0.1 M), NAD⁺ (2 mM) and the enzyme solution were added to each buffer, the reaction was allowed to proceed at 30°C for 1 minute, and the increase in absorbance at the wavelength 340 nm was measured. The oxidation activity measurements were performed within the pH range of 6 to 12 using, as the buffers, 100 mM phosphate buffers (pH 6 to 8), 100 mM Tris hydrochloride buffers (pH 8 to 9), 100 mM carbonate buffers (pH 9 to 11) and 100 mM glycine-NaOH buffers (pH 10 to 12). As a result, the optimum pH for the oxidation was found to be 10.0 to 12.0.

### (Example 6) Substrate specificity of ROB

Using the purified ROB obtained in Example 1, oxidation activity measurements were carried out by adding NAD⁺ (2 mM), each compound (10 mM) shown in Table 2 and the enzyme solution to 100 mM phosphate buffer (pH 8.0), the reaction was allowed to proceed at 30°C for 1 minute, and the increase in absorbance at the wavelength 340 nm was measured. The relative activity data are summarized in Table 2, with the activity against 2,3-butanediol being taken as 100.

Using the purified ROB obtained in Example 1, reduction activity measurements were carried out by adding NADH (0.167 mM), each compound (1 mM) shown in Table 3 and the enzyme solution to 100 mM phosphate buffer (pH 5.5), the reaction was allowed to proceed at 30°C for 3 minutes, and the decrease in absorbance at the wavelength 340 nm was measured. The relative activity data are summarized in Table 3, with the activity against 2,3-butanedione being taken as 100.

**[Table 2]**

| Substrate | Relative activity (%) |
|---|---|
| 2,3-butanediol | 100 |
| 1,2-propanediol | 57.9 |
| 1,2-butanediol | 8.2 |
| 1,2-pentanediol | 4.4 |
| 1,3-butanediol | 0.5 |
| 1,4-pentanediol | 3.3 |
| 3-chloro-1,2-propanediol | 3.9 |
| Methanol | 0 |
| Ethanol | 0 |
| 1-propanol | 0 |
| 1-butanol | 0 |
| 2-propanol | 5.1 |
| 2-butanol | 40.4 |
| 2-pentanol | 58.8 |
| 3-pentanol | 3.3 |
| 2-octanol | 59.6 |
| Acetoin | 0.4 |
| 1-chloro-2-propanol | 7.7 |
| 1-phenyl ethanol | 6.8 |
| 1-phenyl-2-propanol | 43.0 |
| Methyl lactate | 0.9 |
| Ethyl lactate | 1.6 |

**[Table 3]**

| Substrate | Relative activity (%) |
|---|---|
| 2,3-butanedione | 100 |
| 1-chloro-3-hydroxyacetone | 94.3 |
| Chloroacetone | 60.4 |
| Hydroxyacetone | 17.9 |
| 2-butanone | 0.9 |
| 2-pentanone | 2.8 |
| 2-hexanone | 2.8 |
| 2-heptanone | 3.8 |
| 2-octanone | 2.8 |
| Methyl pyruvate | 136 |
| Ethyl pyruvate | 176 |
| Methyl acetoacetate | 65.1 |
| Ethyl 2-chloroacetoacetate | 58.5 |
| Ethyl 4-chloroacetoacetate | 21.7 |

### (Example 7) Stereoselectivity of ROB

Using the purified ROB obtained in Example 1, oxidation activity measurement were carried out using, as substrates, optical isomers of each of 2-butanol, 2-pentanol, 1,2-propanediol, 2,3-butanediol and 3-chloro-1,2-propanediol. NAD⁺ (2 mM), each compound (10 mM) and the enzyme solution were added to 100 mM phosphate buffer (pH 8.0), the reaction was allowed to proceed at 30°C for 1 minute, and the increase in absorbance at the wavelength 340 nm. The relative activity data are summarized in Table 4, with the activity against (2S,3S)-2,3-butanediol being taken as 100.

**[Table 4]**

| Substrate | Relative activity (%) |
|---|---|
| (2S,3S)-2,3-butanediol | 100 |
| (2R,3R)-2,3-butanediol | 0.6 |
| meso-2,3-butanediol | 38.9 |
| (S)-2-butanol | 44.2 |
| (R)-2-butanol | 3.2 |
| (S)-2-pentanol | 58.9 |
| (R)-2-pentanol | 2.9 |
| (S)-1,2-propanediol | 16.7 |
| (R)-1,2-propanediol | 8.0 |
| (S)-3-chloro-1,2-propanediol | 0.1 |
| (R)-3-chloro-1,2-propanedio | 3.5 |

### (Example 8) Behaviors of ROB against reagents

(2S,3S)-2,3-butanediol oxidation activity measurements were carried out in the presence of various reagents shown in Table 5. NAD⁺ (2 mM), (2S,3S)-2,3-butanediol (0.1 M) and the enzyme solution were added to 100 mM phosphate buffer (pH 8.0), the reaction was allowed to proceed at 30°C for 1 minute, and the increase in absorbance at the wavelength 340 nm was measured. The relative activity data are summarized in Table 5, with the activity under reagent-free conditions being taken as 100.

**[Table 5]**

| Inhibitor | Concentration | Relative activity |
|---|---|---|
| | (mM) | (%) |
| None | - | 100 |
| p-chloromercuribenzoate | 1.0 | 84 |
| Phenylmethylsulfonyl fluoride | 1.0 | 75 |
| N-ethylmaleimide | 1.0 | 93 |
| EDTA | 1.0 | 95 |
| Sodium acetate | 1.0 | 96 |
| MgSO₄ | 1.0 | 91 |
| CaCl₂ | 1.0 | 86 |
| BaCl₂ | 1.0 | 56 |
| CoCl₂ | 1.0 | 66 |
| MnCl₂ | 1.0 | 87 |
| CuSO₄ | 1.0 | 81 |
| ZnSO₄ | 1.0 | 88 |
| AgNO₃ | 1.0 | 30 |
| HgCl₂ | 1.0 | 0 |
| HgCl₂ | 0.1 | 41 |

### (Example 9) Cloning of ROB

Using the purified ROB obtained in Example 1, the N terminal amino acid sequence thereof was analyzed with a model ABI492 protein sequencer (product of Applied Biosystems). Further, the purified ROB was denatured in the presence of 8M urea, the product was digested with Achromobacter-derived lysylendopeptidase, and the amino acid sequences of the peptide fragments obtained were analyzed. Considering a DNA sequence estimated from these amino acid sequences, primer 1 (5'-AARGAYGGNTTYGAYATHGC-3': SEQ ID NO:3) and primer 2 (5'-GCYTGNCCYGTDATRTARTC-3': SEQ ID NO:4) were synthesized. An ExTaq buffer (50 µl) containing the two primers (primer 1 and primer 2; each 50 pmol), an Ochrobactrum sp. KNKc71-3 strain-derived chromosomal DNA (14 ng), dNTPs (each 10 nmol) and ExTaq (2.5 U; product of Takara Shuzo) was prepared, 30 cycles of thermal denaturation (95°C, 1 minute), annealing (65°C, 1 minute) and elongation reaction (72°C, 1 minute) were carried out and, after cooling to 4°C, an amplified DNA was confirmed by agarose gel electrophoresis. The Ochrobactrum sp. KNKc71-3 strain-derived chromosomal DNA used in this reaction was prepared by the bacterial genomic DNA minipreparation method described in Bunshi Seibutsugaku Jikken Purotokoru (Protocols for Experiments in Molecular Biology) 1 (published by Maruzen), page 36.

The amplified DNA was subcloned in pT7Blue Vector (product of Novagen) and the base sequence thereof was determined. As a result, the amplified DNA was composed of 628 bases, exclusive of the primer sequences. That sequence is shown under SEQ ID NO:5. Hereinafter, this sequence is referred to as "core sequence".

Based on the base sequence close to the 5' side of the core sequence, primer 3 having a sequence (5'-ATCGGCTGAGAATGTGGACGCCTT-3': SEQ ID NO: 6) complementary to that sequence was synthesized and, based on the base sequence close to the 3' side, primer 4 (5'-TATGTCGACGGCATTGCGCTTGGT-3': SEQ ID NO:7) was synthesized. For preparing a template for inverse PCR, the Ochrobactrum sp. KNKc71-3 strain-derived chromosomal DNA was first digested with the restriction enzyme ApaLI, and the digest was self-circularized using T4 DNA ligase. An ExTaq buffer (50 µl) containing this self-circularization product (100 ng), the two primers (primer 3 and primer 4; each 50 pmol), dNTPs (each 10 nmol) and ExTaq (2.5 U; product of Takara Shuzo) was prepared, 30 cycles of thermal denaturation (95°C, 1 minute), annealing (65°C, 1 minute) and elongation reaction (72°C, 5 minutes) were carried out and, after cooling to 4°C, an amplified DNA was confirmed by agarose gel electrophoresis.

The amplified DNA was subcloned in pT7Blue Vector (product of Novagen) and the base sequence thereof was determined. Based on this result and the result obtained from the core sequence, the whole base sequence of the gene coding for the Ochrobactrum sp. KNKc71-3 strain-derived ROB was determined. The whole base sequence of the ROB-encoding gene is shown under SEQ ID NO:2, and the estimated amino acid sequence encoded by that gene is shown under SEQ ID NO:1.

### (Example 7.0) Construction of ROB gene-containing recombinant vector

For the expression of ROB in Escherichia coli, a recombinant vector to be used for transformation was constructed. First, a double-stranded DNA with an NdeI site added to the initiation codon site of the ROB gene and a new termination codon and an EcoRI site added just behind the termination codon was obtained in the following manner.

Based on the base sequence determined in Example 9, primer 5 (5'-AGGAAAGGATGACATATGTCGGCTAACACCAAGGT-3': SEQ ID NO:8) containing an NdeI site added to the initiation codon site of the ROB gene and primer 6 (5'-GCGGAATTCTCAGCGGTAAACGATACCGC-3': SEQ ID NO:9) containing an EcoRI site added just behind the termination codon of the ROB gene were synthesized. An ExTaq buffer (50 µl) containing the two primers (primer 5 and primer 6; each 50 pmol), the Ochrobactrum sp. KNKc71-3 strain-derived chromosomal DNA (14 ng), dNTPs (each 10 nmol) and ExTaq (2.5 U; product of Takara Shuzo) was prepared, 30 cycles of thermal denaturation (95°C, 1 minute), annealing (65°C, 1 minute) and elongation reaction (72°C, 1 minute) were carried out and, after cooling to 4°C, an amplified DNA was confirmed by agarose gel electrophoresis. This amplified fragment was digested with NdeI and EcoRI and the digest was inserted into the plasmid pUCNT constructible by those skilled in the art according to the method disclosed in WO 94/03613 into the NdeI and EcoRI sites downstream from the lac promoter; a recombinant vector, pNTOB, was thus obtained.

### (Example 11) Addition of Shine-Dalgarno sequence to site upstream of ROB gene

For high-level expression of the ROB gene in Escherichia coli, a plasmid containing Escherichia coli Shine-Dalgarno sequence (9 bases) added to the plasmid pNTOB prepared in Example 10 at a site upstream of the initiation codon was obtained in the following manner.

First, a plasmid, pUCT, containing T in lieu of the G in the NdeI site of the Escherichia coli expression vector pUCNT used in Example 10 was constructed by the PCR method. Then, based on the base sequence determined in Example 9, primer 7 (5'-GCCGAATTCTAAGGAGGTTAACAATGTCGGCTAACACCAAGGT-3': SEQ ID NO:10) containing the Escherichia coli Shine-Dalgarno sequence (9 bases) at a site 5 bases upstream from the initiation codon of the ROB gene and further containing an EcoRI site added just before the Shine-Dalgarno sequence and primer 8 (5'-GCGGGATCCTCAGCGGTAAACGATACCGC-3': SEQ ID NO:11) containing a BamHI site added just behind the termination codon of the ROB gene were synthesized.

An ExTaq buffer (50 µl) containing the two primers (primer 7 and primer 8; each 50 pmol), the Ochrobactrum sp. KNKc71-3 strain-derived chromosomal DNA (14 ng), dNTPs (each 10 nmol) and ExTaq (2.5 U; product of Takara Shuzo) was prepared, 30 cycles of thermal denaturation (95°C, 1 minute), annealing (65°C, 1 minute) and elongation reaction (72°C, 1 minute) were carried out and, after cooling to 4°C, an amplified DNA was confirmed by agarose gel electrophoresis. This amplified fragment was digested with EcoRI and BamHI and the digest was inserted into the above-mentioned plasmid pUCT at the EcoRI and BamHI sites downstream from the lac promoter; a recombinant vector, pTSOB, was thus obtained. The method of construction and the structure of pTSOB are shown in Fig. 1.

### (Example 12) Construction of recombinant vector simultaneously containing ROB gene and glucose dehydrogenase gene

A double-stranded DNA containing the Escherichia coli Shine-Dalgarno sequence (9 bases) added 5 bases upstream from the initiation codon of glucose dehydrogenase (hereinafter referred to as GDH) gene derived from the Bacillus megaterium IAM 1030 strain and further containing a BamHI site added just before that sequence and a PstI site added just behind the termination codon was obtained in the following manner.

Based on the base sequence information about the GDH gene, primer 9 (5'-GCCGGATCCTAAGGAGGTTAACAATGTATAAADATTTAGAAGG-3': SEQ ID NO:12) containing the Escherichia coli Shine-Dalgarno sequence (9 bases) added 5 bases upstream from the initiation codon of the structural gene for GDH and a BamHI site added just before that sequence and primer 10 (5'-GCGCTGCAGTTATCCGCGTCCTGGTTGGA-3': SEQ ID NO:13) containing a PstI site added just behind the termination codon were synthesized in the conventional manner.

Using these two primers, with the plasmid pGDK1 (Eur. J. Biochem., 186, 389 (1989)) as a template, a double-stranded DNA was synthesized by PCR. The DNA fragment obtained was digested with BamHI and PstI, and the digest was inserted into the pTSOB constructed in Example 11 at the BamHI and PstI sites; a recombinant vector pTSOBG1 was thus obtained. The method of construction and the structure of pTSOBG1 are shown in Fig. 1.

### (Example 13) Production of recombinant Escherichia coli

The recombinant vector pTSOB obtained in Example 11 and the recombinant vector pTSOBG1 obtained in Example 12 were used to transform Escherichia coli HB101 (product of Takara Shuzo) to give recombinant Escherichia coli HB101(pTSOB) and HB101(pTSOBG1), respectively. The thus-obtained transformant, namely Escherichia coli HB101(pTSOB) has been deposited as of November 30, 2005 with the National Institute of Advanced Industrial Science and Technology International Patent Organism Depositary (IPOD: zip code 305-8566, Chuo No. 6 Higashi 1-1-1 Tsukuba City, Ibaraki Prefecture) under the accession number FERM BP-10461.

### (Example 14) Expression of ROB in recombinant Escherichia coli

The recombinant Escherichia coli HB101(pTSOB) and HB101(pTSOBG1) obtained in Example 13 and Escherichia coli HB101(pUCT), a transformant as transformed with the vector plasmid alone, were respectively cultured on 2xYT medium (1.6% Bacto tryptone, 1.0% Bacto yeast extract, 0.5% NaCl, pH 7.0) containing 100 µg/ml ampicillin. Cells were collected from each culture, suspended in 100 mM phosphate buffer (pH6.5) and disrupted by sonication using a model UH-50 ultrasonic homogenizer (product of SMT) to give a cell-free extract. The (2S,3S)-2,3-butanediol oxidation activities of the thus-obtained cell-free extracts are shown in Table 6, and the 2,3-butanedione reduction activities and GDH activities thereof are shown in Table 7.

The 2,3-butanedione reduction activity measurements were carried out by adding 2,3-butanedione (10 mM), NADH (0.167 mM) and the enzyme solution to 100 mM phosphate buffer (pH 5.5), the reaction was allowed to proceed at 30°C for 3 minutes and then the decrease in absorbance at the wavelength 340 nm was measured. The GDH activity measurements were carried out by adding glucose (0.1 M), NADP⁺ (2 mM) and the enzyme to 1 M Tris hydrochloride buffer (pH 8.0) and measuring the increase in absorbance at the wavelength 340 nm at 25°C.

**[Table 6]**

| Strain | (2S,3S)-2,3-butanediol oxidation activity (U/mg) |
|---|---|
| *E. coli* HB101 (pUCT) | 0.1 |
| *E. coli* HB101 (pTSOB) | 15.2 |

**[Table 7]**

| Strain | 2,3-butanedione reduction activity (U/mg) | GDH activity (U/mg) |
|---|---|---|
| *E. coli* HB101 (pUCT) | 0.3 | >0.01 |
| *E. coli* HB101 (pTSOBG1) | 38.9 | 49.2 |

### (Example 15) Synthesis of (R)-1,3-butanediol using recombinant Escherichia coli with ROB gene introduced therein

To 1 ml of the recombinant Escherichia coli HB101 (pTSOB)-derived cell-free extract obtained in Example 14 were added 10 mg of racemic 1,3-butanediol and 50 mg of NAD⁺, and the mixture was shaken at 30°C for 20 hours. After completion of the reaction, the reaction mixture was saturated with ammonium sulfate, ethyl acetate was added for extraction, and the residual 1,3-butanediol and the product 4-hydoxy-2-butanone were assayed by capillary gas chromatography (column: HP-5, 30 m x 0.32 mm I.D., detection: FID, column temperature: 60°C, injection temperature: 150°C, detection temperature: 150°C, carrier gas: helium (150 kPa), split ratio: 100/1). The optical purity of the residual 1,3-butanediol was determined by trifluoroacetylating the hydroxyl groups of the 1,3-butanediol and subjecting the product to gas chromatography (column; G-PN, 30 m x 0.32 mm I.D. (product of Tokyo Kasei Kogyo), detection: FID, column temperature: 70°C, injection temperature: 150°C, detection temperature: 150°C, carrier gas: helium (60 kPa), split ratio: 100/1).

As a result, 6.0 mg of 4-hydroxy-2-butanone was formed, the residual 1, 3-butanediol weighed 3.9 mg and occurred in the R form, with an optical purity of 100% e.e.

### (Example 16) Synthesis of (S)-3-chloro-1,2-propanediol using recombinant Escherichia coli with ROB gene introduced therein

To 1 ml of the recombinant Escherichia coli HB101(pTSOB)-derived cell-free extract obtained in Example 14 were added 5 mg of racemic 3-chloro-1,2-propanediol, 10 U of NADH oxidase (derived from Streptococcus mutans NCIMB 11723) and 1 mg of NAD⁺, and the mixture was shaken at 20°C for 5 hours. After completion of the reaction, the reaction mixture was saturated with ammonium sulfate, ethyl acetate was added for extraction, and the residual 3-chloro-1,2-propanediol was assayed by capillary gas chromatography (column: HP-5, 30 m × 0.32 mm I.D., detection: FID, column temperature: 90°C, injection temperature: 150°C, detection temperature: 150°C, carrier gas: helium (150 kPa), split ratio: 100/1). The optical purity of the residual 3-chloro-1,2-propanediol was determined by trifluoroacetylating the hydroxyl groups of the 3-chloro-1,2-propanediol and subjecting the product to gas chromatography (column; G-PN, 30 m x 0.32 mm I.D. (product of Tokyo Kasei Kogyo), detection: FID, column temperature: 90°C, injection temperature: 150°C, detection temperature: 150°C, carrier gas: helium (60 kPa), split ratio: 100/1). As a result, the residual 3-chloro-1,2-propanediol weighed 2.1 mg and occurred in the S form, with an optical purity of 100% e.e.

### (Example 17) Synthesis of (S)-1,3-butanediol using recombinant Escherichia coli simultaneously expressing ROB and glucose dehydrogenase

To 1 ml of the recombinant Escherichia coli HB101(pTSOBG1)-derived cell-free extract obtained in Example 14 were added 40 mg of glucose, 1 mg of NAD⁺ and 10 mg of 4-hydroxy-2-butanone, and the mixture was shaken at 30°C for 10 hours. After completion of the reaction, the reaction mixture was saturated with ammonium sulfate, ethyl acetate was added for extraction, and the product 1,3-butanediol was assayed under the same conditions as in Example 15. As a result, the 1,3-butanediol formed weighed 9.3 mg and occurred in the S form, with an optical purity of 100% e.e.

### (Example 18) Synthesis of (S)-2-pentanol using recombinant Escherichia coli simultaneously expressing ROB and glucose dehydrogenase

To 1 ml of the recombinant Escherichia coli HB101(pTSOBG1)-derived cell-free extract obtained in Example 14 were added 40 mg of glucose, 1 mg of NAD⁺ and 10 mg of 2-pentanone, and the mixture was shaken at 30°C for 10 hours. After completion of the reaction, the reaction mixture was saturated with ammonium sulfate, ethyl acetate was added for extraction, and the product 2-pentanol was assayed by capillary gas chromatography (column: HP-5, 30 m x 0.32 mm I.D., detection: FID, column temperature: 30°C, injection temperature: 150°C, detection temperature: 150°C, carrier gas: helium (150 kPa), split ratio: 100/1). The optical purity of the product 2-pentanol was determined by trifluoroacetylating the hydroxyl group of 2-pentanol and subjecting the product to gas chromatography (column; G-PN, 30 m x 0.32 mm I.D., detection: FID, column temperature: 30°C, injection temperature: 150°C, detection temperature: 150°C, carrier gas: helium (130 kPa), split ratio: 100/1).

As a result, the 2-pentanol formed weighed 9.9 mg and occurred in the S form, with an optical purity of 98.7% e.e.

## Claims

1. A polypeptide having the physicochemical properties shown below under (1) to (2):
(1) Actions:
(a) Acting on (2S,3S)-2,3-butanediol to form (S)-acetoin in the presence of oxidized-form nicotinamide adenine dinucleotide (hereinafter abbreviated as NAD⁺) as a coenzyme;
(b) Reducing 2,3-butanedione to form (2S,3S)-2,3-butanediol in the presence of reduced-form nicotinamide adenine dinucleotide (hereinafter abbreviated as NADH) as a coenzyme;
(2) Substrate specificities:
(a) Utilizing NAD⁺ as a coenzyme in oxidation reactions; also utilizing NADH as a coenzyme in reduction reactions;
(b) Oxidizing not only the (S)-configuration hydroxyl group in (2S,3S)-2,3-butanediol among the three isomers of 2,3-butanediol but also the (S)-configuration hydroxyl group in meso-2,3-butanediol.

2. The polypeptide according to Claim 1,
further having the physicochemical property shown below under (3):
(3) Substrate specificities: Selectively oxidizing the (R)-configuration hydroxyl group in racemic 3-chloro-1,2-propanediol and allowing (S)-3-chloro-1,2-propanediol to continue to exist.

3. The polypeptide according to Claim 1 or 2,
further having the physicochemical properties shown below under (4) to (6):
(4) Molecular weight: about 30,000 as determined by sodium dodecyl sulfate-polyacrylamide gel electrophoresis;
(5) Optimum pH for oxidation: from 10.0 to 12.0;
(6) Optimum pH for reduction: from 5.0 to 5.5.

4. The polypeptide according to any of Claims 1 to 3,
which is produced by a microorganism belonging to the genus Ochrobactrum.

5. The polypeptide according to Claim 4,
wherein the microorganism belonging to the genus Ochrobactrum is an Ochrobactrum sp.

6. A polypeptide defined below under (a), (b) or (c):
(a) a polypeptide comprising the amino acid sequence shown in the sequence listing under SEQ ID NO:1;
(b) a polypeptide comprising an amino acid sequence derived from the amino acid sequence shown in the sequence listing under SEQ ID NO:1 by substitution, insertion, deletion and/or addition of one or a plurality of amino acids, said polypeptide having an enzymatic activity of selectively oxidizing the (R)-configuration hydroxyl group of racemic 3-chloro-1,2-propanediol and allowing (S)-3-chloro-1,2-propanediol to continue to exist;
(c) a polypeptide comprising an amino acid sequence having at least 80% sequence homology to the amino acid sequence shown in the sequence listing under SEQ ID NO:1, said polypeptide having an enzymatic activity of selectively oxidizing the (R)-configuration hydroxyl group in racemic 3-chloro-1,2-propanediol and allowing (S)-3-chloro-1,2-propanediol to continue to exist.

7. A DNA coding for the polypeptide according to any of Claims 1 to 6.

8. A DNA defined below under (a), (b) or (c):
(a) a DNA comprising the base sequence shown in the sequence listing under SEQ ID NO:2;
(b) a DNA capable of hybridizing with a DNA comprising a base sequence complementary to the base sequence shown in the sequence listing under SEQ ID NO:2 under stringent conditions, said DNA coding for a polypeptide having an enzymatic activity of selectively oxidizing the (R) -configuration hydroxyl group in racemic 3-chloro-1,2-propanediol and allowing (S)-3-chloro-1,2-propanediol to continue to exist;
(c) a DNA comprising a base sequence having at least 80% sequence homology to the base sequence shown in the sequence listing under SEQ ID NO:2, said DNA coding for a polypeptide having an enzymatic activity of selectively oxidizing the (R)-configuration hydroxyl group in racemic 3-chloro-1,2-propanediol and allowing (S)-3-chloro-1,2-propanediol to continue to exist.

9. An expression vector comprising the DNA according to Claim 7 or 8.

10. The expression vector according to Claim 9,
which is the plasmid pTSOB capable of being isolated from Escherichia coli HB101 (pTSOB) (FERM BP-10461).

11. A transformant obtained by transforming host cells with the expression vector according to Claim 9 or 10.

12. The transformant according to Claim 11,
wherein the host cell is Escherichia coli.

13. The transformant according to Claim 11,
which is Escherichia coli HB101 (pTSOB) (FERM BP-10461).

14. A method of producing an optically active alcohol, comprising the step of:
allowing the polypeptide according to any of Claims 1 to 6 or the culture product of the transformant according to any of Claims 11 to 13 to act on a racemic alcohol to thereby oxidize an alcohol having one configuration and allow an alcohol having the other configuration to continue to exist.

15. A method of producing an optically active alcohol, comprising the step of:
allowing the polypeptide according to any of Claims 1 to 6 or the culture product of the transformant according to any of Claims 11 to 13 to act on a carbonyl compound.
